# EUROPÄISCHE PATENTSCHRIFT

(11) **EP 3 215 206 B1**
(45) Veröffentlichungstag und Bekanntmachung des Hinweises auf die Patenterteilung: **12.02.2020**
(21) Anmeldenummer: 15777614.7
(22) Anmeldetag: 11.09.2015
(51) Int. Cl.: A61M 15/06, A61M 11/04, A24F 47/00, F24V 30/00

(54) **HEIZVORRICHTUNG FÜR EINE RAUCHFREIE ZIGARETTE**
HEATING DEVICE FOR A SMOKE-FREE CIGARETTE
DISPOSITIF CHAUFFANT DE CIGARETTE ÉLECTRONIQUE

(30) Priorität: 07.11.2014 DE 102014116258
(43) Veröffentlichungstag der Anmeldung: 13.09.2017
(73) Patentinhaber: S.A.S.C. AG, 6340 Baar (CH)
(72) Erfinder: KIESLICH, Dirk, 58840 Plettenberg (DE)
(74) Vertreter: Patentanwälte Dörner & Kötter PartG mbB
(86) Internationale Anmeldenummer: PCT/EP2015/070831
(87) Internationale Veröffentlichungsnummer: WO 2016/071027

(56) Entgegenhaltungen:
- WO-A1-2013/128176
- WO-A2-2014/045024
- DE-A1-102011 111 999
- DE-C- 626 744
- US-A1- 2005 016 549
- US-A1- 2014 069 424

## Beschreibung

Die Erfindung betrifft eine Heizvorrichtung für eine rauchfreie Zigarette. Außerdem betrifft die Erfindung die Verwendung einer Heizvorrichtung in einer rauchfreien Zigarette sowie eine rauchfreie Zigarette mit einer Heizvorrichtung.

Handelsübliche Zigaretten bestehen meist aus in Papier oder einem Tabakblatt, gewickeltem Tabak sowie einem am Mundstück angebrachten Filterelement. Durch Anzünden des vorderen Endes der Zigarette verglüht und verschwelt der Tabak und setzt Nikotin frei, welcher durch den vom Raucher zu inhalierenden Rauch mitgerissen wird. Bei der Verbrennung des Tabaks werden neben Nikotin auch andere Substanzen freigesetzt bzw. erzeugt, die häufig gesundheitsschädlich sind, so zum Beispiel Teer, Arsen- und Cadmiumhaltige Verbindungen sowie kanzerogene Verbindungen wie Hydrazin, Chrysen, Formaldehyd, Nietrosamine und ähnliches.

Zur Vermeidung dieser Risiken sind so genannte rauchfreie Zigaretten bekannt. Hierbei handelt es sich um Produkte, bei denen der Tabak nur erwärmt wird, ohne verbrannt zu werden, so dass, zwar in geringen Dosen, das gewünschte Nikotin freigesetzt, die Entstehung gesundheitsschädlicher Stoffe jedoch vermieden wird. Ein weiterer Ansatz für eine rauchfreie Zigarette ist in der DE 103 21 379 A1 beschrieben. Bei dieser rauchfreien Zigarette wird mit Hilfe einer elektrischen Heizeinrichtung Luft erwärmt und die erwärmte Luft durch ein Nikotindepot geleitet. Das Nikotin soll hierbei in geringen Maßen durch die erwärmte Luft freigesetzt und von dem Benutzer inhaliert werden. In einer weiteren Ausführungsform wird das Nikotin aus einem wieder auffüllbaren Depot direkt durch eine elektrisch betriebene Heizwendel verdampft und durch einen Filter, der das Mundstück bildet, vom Benutzer inhaliert (vgl. NicStick®-Zigarette).

Diese sogenannten "elektrischen Zigaretten" erfüllen zwar die an sie gestellten Anforderungen in Bezug auf Zuverlässigkeit, Geschmack usw. Sie weisen jedoch den Nachteil auf, dass ihre Heizquelle kurz vor Rauchbeginn erwärmt werden muss, und zwar durch Zuführung elektrischer Energie über Heizspiralen oder Verdampfer. Oder es muss die Aufladung durch eine wieder aufladbare Batterie erfolgen. Somit ist zur Funktion der rauchfreien Zigarette die Zuführung von Energie erforderlich. Auch können die Zigaretten nicht problemlos entsorgt werden. Dies findet seine Ursache in der Verwendung der elektrischen Heizeinrichtung, die im Falle der Entsorgung dem Restmüll zuzuführen ist. Da dieses Erfordernis bei handelsüblichen Zigaretten nicht besteht, weil es sich bei ihren Bestandteilen, das sind Papier oder Tabakblatt einerseits und gewickeltem Tabak andererseits, um verrottende Materialien handelt, muss beim Umsteigen von handelsüblichen Zigaretten auf elektrische Zigaretten gleichzeitig ein Umdenken beim Benutzer erfolgen, was jedoch vereinzelt nicht der Fall ist.

Aus der WO2013/128176 A1 und der WO2014/045024 A2 sind Heizvorrichtungen bekannt, die Flüssigkeitsbehälter und Reaktionselemente aufweisen, die mit Stoffen gefüllt sind, deren Vermischung eine exotherme Reaktion auslöst.

Der Erfindung liegt die Aufgabe zugrunde, eine Heizvorrichtung für eine rauchfreie Zigarette zu schaffen, die eine zuverlässige Abgabe des Nikotins ermöglicht und gleichzeitig eine Heizeinrichtung aus verrottendem bzw. wiederverwertbarem Material bereitstellt, die ohne Zuführung externer Energie funktionsfähig ist. Gemäß der Erfindung wird diese Aufgabe durch eine Heizvorrichtung für eine rauchfreie Zigarette mit den Merkmalen des Patentanspruchs 1 gelöst.

Mit der Erfindung ist eine Heizvorrichtung für eine rauchfreie Zigarette geschaffen, die eine zuverlässige Abgabe des Nikotins ermöglicht und gleichzeitig eine Heizeinrichtung aus verrottendem bzw. wiederverwertbarem Material bereitstellt, weil lediglich zwei gesundheitsunschädliche und biologisch unbedenkliche Stoffe miteinander reagieren. Durch die Vermischung der beiden Stoffe entsteht eine exotherme Reaktion infolge einer chemischen Reaktion zwischen den Stoffen, bei der Energie in Form von Wärme freigesetzt wird. Hierbei ist eine Temperatur erreichbar, die der Temperatur des eingeatmeten Gemischs beim Rauchen handelsüblicher Zigaretten entspricht. Über die Größe des Flüssigkeitsbehälters sowie die Menge der Stoffe ist der Zeitraum der Reaktion einstellbar. Mit der erfindungsgemäßen Heizeinrichtung können daher die Eigenschaften und das Empfinden einer handelsüblichen Zigarette nachgebildet werden, ohne deren gesundheitlichen Nachteile zu beinhalten. Die Verwendung von Calciumchlorid als Füllung für das Reaktionselement gewährleistet dabei eine zuverlässige exotherme Reaktion beim Aufeinandertreffen mit dem Wasser. Dadurch ist die Funktionssicherheit der erfindungsgemäßen Heizvorrichtung sichergestellt.

In Ausgestaltung der Erfindung ist der Flüssigkeitsbehälter mit Wasser gefüllt. Der wesentliche Vorteil bei der Verwendung von Wasser für die exotherme Reaktion besteht darin, dass es einerseits einfach und ohne Risiken verarbeitbar ist, andererseits im Hinblick auf Umweltschutzkriterien unbedenklich ist.

Andere Weiterbildungen und Ausgestaltungen der Erfindung sind in den übrigen Unteransprüchen angegeben. Ausführungsbeispiele der Erfindung sind in den Zeichnungen dargestellt und werden nachfolgend im Einzelnen beschrieben. Es zeigen:

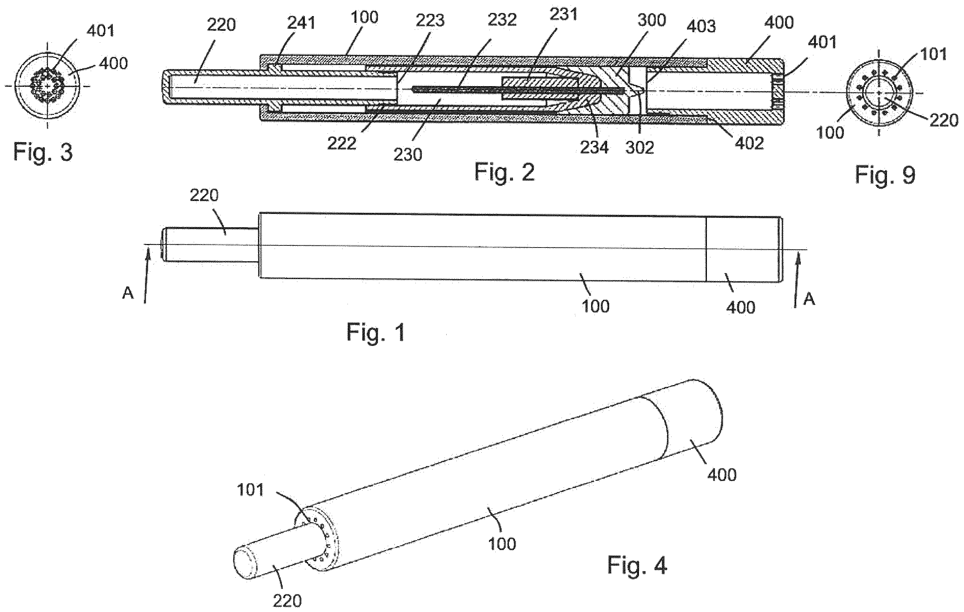

- Figur 1: die Ansicht einer rauchfreien Zigarette mit einer erfindungsgemäßen Heizvorrichtung in nicht aktiviertem Zustand;
- Figur 2: den Schnitt entlang der Linie A-A in Figur 1;
- Figur 3: die Ansicht von rechts auf die in Figur 1 dargestellte rauchfreie Zigarette;
- Figur 4: die perspektivische Darstellung der in Figur 1 dargestellten rauchfreien Zigarette;
- Figur 5: die Ansicht der in Figur 1 dargestellten rauchfreien Zigarette in aktiviertem Zustand;
- Figur 6: den Schnitt entlang der Linie D-D in Figur 5;
- Figur 7: die perspektivische Darstellung der in Figur 5 dargestellten rauchfreien Zigarette;

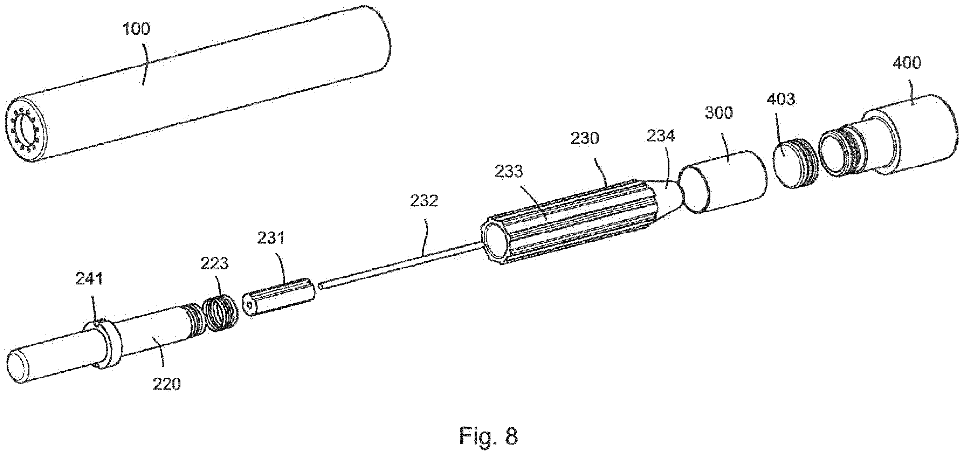

- Figur 8: die explosionsartige Darstellung der rauchfreien Zigarette nach den Figuren 4 und 7 sowie deren Bauteile;
- Figur 9: die Ansicht von links auf die in Figur 1 dargestellte rauchfreie Zigarette.

Im Ausführungsbeispiel ist eine rauchfreie Zigarette dargestellt, die einen hülsenförmigen Körper 100 umfassen. In dem Körper 100 sind eine Heizvorrichtung 200 mit einem Reaktionspartner und ein Depot 300 angeordnet. Die rauchfreie Zigarette weist ein dem Depot 300 in Ansaugrichtung nachgeordnetes Mundstück 400 auf sowie eine Aktivierungseinrichtung 500 zur Aktivierung der Reaktion und / oder Freigabe der Inhalationsstoffe. Die rauchfreie Zigarette ist nachfüllbar.

Der Körper 100 ist im Ausführungsbeispiel aus Pappe hergestellt. In Abwandlung des Ausführungsbeispiels können auch andere Materialien, wie beispielsweise verrottender Kunststoff oder Metall, zum Beispiel Aluminium, zur Anwendung kommen. Der Körper 100 ist an seinen beiden Enden offen ausgebildet. An seinem einen Ende ist ein zylindrischer Absatz vorgesehen, der zum Aufstecken des Mundstücks 400 dient. Im mittleren Bereich des Körpers 100 ist zudem eine Aufnahme ausgebildet, in die das Depot 300 einsetzbar ist.

Das Depot 300 kann verschiedene dreidimensionale Formen aufweisen.

Im Ausführungsbeispiel ist das Mundstück 400 in den Zigarettenkörper 100 eingesteckt. Es ist hierfür außen mit einem Anschlag 402 versehen. Das mit den Austrittsöffnungen 401 versehene Mundstück 400 ist mittels einer Membran 403 dicht verschlossen. Die Membran 403 besteht vorzugsweise aus Aluminiumfolie.

Auf der dem Mundstück 400 abgewandten Seite ist der Zigarettenkörper 100 mit Eintrittsöffnungen 101 versehen. In dem Zigarettenkörper 100 ist ein verschiebbarer Flüssigkeitsbehälter 220 angeordnet, der mit Wasser gefüllt ist und der aus wärmeleitendem Kunststoff oder Metall besteht, wobei bevorzugt korrosionsbeständiges Eisen bzw. Aluminium zum Einsatz kommt. Der Flüssigkeitsbehälter 220 weist auf seinem Umfang einen Anschlag 221 auf, der im nicht aktivierten Zustand die Eintrittsöffnungen 101 verschließt. Der Flüssigkeitsbehälter 220 weist auf seiner dem Depot 300 zugewandten Seite eine Dichtung 223 auf, die den Flüssigkeitsbehälter 220 einseitig gasdicht verschließt. Im Ausführungsbeispiel besteht die Dichtung 223 aus Aluminium. Andere Materialien sind ebenfalls möglich.

In dem Zigarettenkörper 100 ist ein Reaktionselement 230 verschiebbar angeordnet. Das Reaktionselement 230 ist nach Art einer einseitig geschlossenen Hülse ausgebildet. Es ist im Ausführungsbeispiel als Formkörper zum Beispiel aus getrocknetem Calciumchlorid oder einem Erdalkalimetall hergestellt gefüllt. Das Reaktionselement 230 hat einen etwas kleineren Außendurchmesser, als der Innendurchmesser des Zigarettenkörpers 100. Das Reaktionselement 230 ist auf seinem Umfang mit axial ausgerichteten Nuten 233 versehen. Auf der dem Depot 300 zugewandten Seite hat das Reaktionselement 230 eine kegelstumpfförmige Spitze 234. Innen ist an dem dem Depot 300 zugewandten Ende ein Profilkörper 231 vorgesehen. Der Profilkörper 231 ist im Ausführungsbeispiel aus Erdalkalimetall, beispielsweise Lithium, Natrium, Kalium oder Calcium hergestellt. Er kann aber auch beispielsweise aus Calciumchlorid (CaCl), Calciumchlorid (CaCl₂), Magnesiumsulfat (MgSO₄), Natriumhydroxid (NaOH) oder Calciumoxid (CaO) hergestellt sein. Der Profilkörper 231 weist auf seiner Mantelfläche in Längsrichtung Nuten auf. Er hat einen Außendurchmesser, der kleiner als der Innendurchmesser des Reaktionselements 230 ist. Der Profilkörper 231 ist auf den Stift 232 aufgesetzt, der sich koaxial zur Längsmittellinie des Reaktionselementes 230 erstreckt. Der Stift 232 durchsetzt den Profilkörper 231 vollständig und erstreckt sich im Ausführungsbeispiel durch die kegelstumpfförmige Spitze des Reaktionselementes 230 bis in das Depot 300. Er kann in die Spitze des Reaktionselements 230 eingesetzt sein oder unmittelbar Bestandteil des Reaktionselements 230 sein. Der Stift 232 besteht aus biologisch abbaubarem, wärmeleitendem Kunststoff oder Metall.

Das Depot 300 weist auf seiner dem Mundstück 400 zugewandten Seite die Aktivierungseinrichtung 500 in Form eines Dorns 502 auf. Es hat auf der dem Mundstück 400 abgewandten Seite eine kegelstumpfförmige Ausnehmung, die die kegelstumpfförmige Spitze 234 des Reaktionselementes 230 aufnimmt.

Das Depot 300 hat im Ausführungsbeispiel einen relativ formstabilen Körper aus gesintertem Material. Alternativ können auch Vliese oder faserige Materialien zur Anwendung kommen. Das Material ist gas- und luftdurchlässig. Das Depot 300 ist mit dem für den jeweiligen Anwendungsfall erforderlichen gasförmigen Medium gefüllt. Im Falle der Benutzung der Vorrichtung als rauchfreie Zigarette ist das Depot 300 mit Nikotin und einem Trägersubstanz gefüllt. Beispiele für geeignete Trägersubstanzen sind Alkohole, Ester, Ether, Aldehyde, Kohlenwasserstoffe und Gemische daraus oder auch Methanol, Ethanol, Propanol, Essigsäureethylester, Diethylether, Acetaldehyd, Aceton, Pentan, Hexan, Octan und Gemische daraus als Trägersubstanz verwendet werden. Auch können ätherische Öle als Trägersubstanz verwendet werden.

Im Ausführungsbeispiel erfolgt die Aktivierung Heizvorrichtung für eine rauchfreie Zigarette durch Zusammendrücken der verschiebbaren Teile.

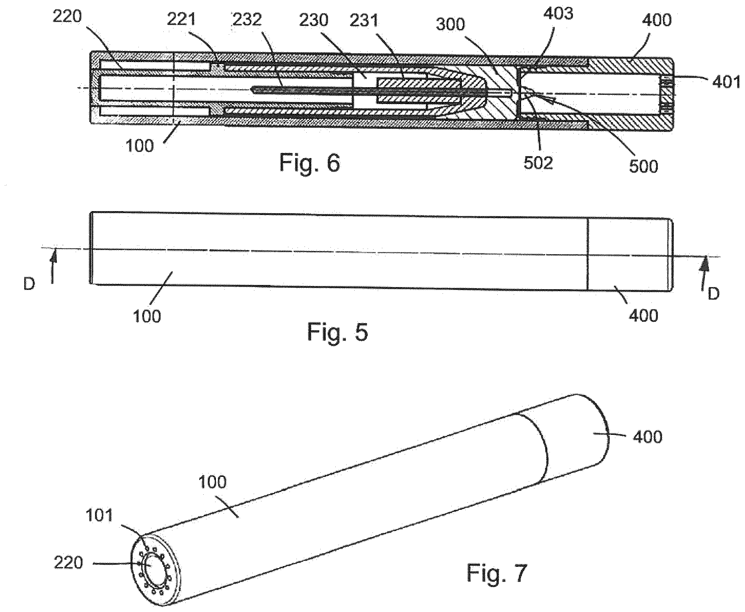

Im Ausführungsbeispiel wird der Flüssigkeitsbehälter 220 in den Zigarettenkörper 100 hineingedrückt. Dadurch kommt die Dichtung 223 mit dem Stift 232 in Kontakt. Durch weiteres Einschieben des Flüssigkeitsbehälters 220 durchstößt der Stift 232 die Dichtung 223. Auf diese Weise tritt das Wasser im Flüssigkeitsbehälter 220 mit dem Profilkörper 231 in Kontakt, wodurch es zu einer exothermen Reaktion kommt. Das Zusammenwirken von des Profilkörpers 231 und des Flüssigkeitsbehälters 220 sowie die dadurch ausgelöste exotherme Reaktion bildet die Heizvorrichtung 200. Der Flüssigkeitsbehälter 220 wird so weit in den Zigarettenkörper 100 hineingedrückt, bis der Anschlag 221 an das stirnseitige Ende des Reaktionselementes 230 anstößt. Bei Ausüben eines weiteren Drucks auf den Flüssigkeitsbehälter 220 wird dann auch das Reaktionselement 230 axial in dem Zigarettenkörper 100 verschoben. Dies bewirkt gleichzeitig ein Verschieben des Depots 300. Dabei tritt die Aktivierungseinrichtung 500 in Form des Dorns 502 durch die Membran 403 im Mundstück 400 (vgl. Figur 6). Dadurch ist das Ansaugen von Luft durch den Benutzer möglich. Diese tritt durch die Eintrittsöffnungen 101 an dem dem Mundstück 400 abgewandten Ende in den Zigarettenkörper 100 ein und streicht entlang des Flüssigkeitsbehälters 220 und dem Reaktionselement 230 entlang dem Depot 300 in das Mundstück 400 und durch die Austrittsöffnungen 401. Beim Vorbeistreichen an dem Reaktionselement 230 wird die Luft aufgrund der durch die Reaktion freigesetzten Wärme auf ca. 50°C erwärmt, wodurch eine zu handelsüblichen Zigaretten vergleichbare Inhalationstemperatur erreicht ist. Mit der warmen Luft wird nach Passieren des Reaktionselementes 230 das Nikotin aus dem Depot 300 gelöst. Die warme Luft tritt danach durch die vom Dorn 502 verursachte Öffnung in der Membran 403 in das Mundstück 400 ein und wird nach Passieren des Mundstücks 400 vom Benutzer inhaliert. Durch das Hineinragen des Stifts 232 bis in das Depot 300 wird zusätzlich eine Erwärmung des Depots 300 von innen erzielt, die zu einer verbesserten Mobilisierung des Nikotins aus dem Depot 300 und damit zu einem verbesserten Wirkungsgrad führt.

In Abwandlung der in den Figuren dargestellten Ausführungsbeispiele besteht auch die Möglichkeit, die Dichtungen und / oder die Membranen aus anderen Materialien herzustellen als Aluminium. Hier kommen insbesondere Kunststofffolien oder Lacke in Betracht. Wesentlich für das ausgewählte Material ist die Eigenschaft, das jeweilige Bauteil dicht abzuschließen, gegen klimatische Schwankungen unempfindlich zu sein und eine ausreichend Sprödigkeit aufzuweisen, um einen zuverlässigen Durchtritt der Stifte und Dorne zu gewährleisten. Die Dichtungen und Membranen schützen vor Inbetriebnahme das gesamte System vor einer Austrocknung und einer Verflüchtigung der Inhaltsstoffe.

Aufgrund der Größe der Flüssigkeitsbehälter beziehungsweise Reaktionselemente und deren Inhalt erfolgt die exotherme Reaktion über einen Zeitraum von 3 bis 5 Minuten, was dem Zeitraum für das Rauchen handelsüblicher Zigaretten entspricht. Folglich ist durch die erfindungsgemäße Heizeinrichtung eine rauchfreie Zigarette geschaffen, die die Eigenschaften und das Empfinden einer handelsüblichen Zigarette nachbildet ohne deren gesundheitlichen Nachteile zu beinhalten. Durch die sehr effiziente Reaktion muss darüber hinaus das Depot mit lediglich 10 mg Nikotin gefüllt werden, um eine im Verhältnis zu handelsüblichen Zigaretten vergleichbare Wirkung über den genannten Zeitraum zu erzielen. Bei den aus dem Stand der Technik bekannten Lösungen sind ca. 18 mg erforderlich. Dabei handelt es sich im Falle eines Verschluckens um eine äußerst gesundheitsgefährliche, wenn nicht tödliche Dosierung.

## Patentansprüche

1. Heizvorrichtung für eine rauchfreie Zigarette, bestehend aus einem Flüssigkeitsbehälter (220), der mit einem Stoff gefüllt ist, und einem Reaktionselement (230), das aus einem Stoff hergestellt ist, wobei eine Vermischung dieser Stoffe eine exotherme Reaktion auslöst und die durch mindestens eine zerstörbare Dichtung (223) voneinander getrennt sind, wobei am Reaktionselement (230) ein Stift (232) vorgesehen ist, der mit der Dichtung (223) zusammenwirkt, **dadurch gekennzeichnet, dass** der Stift (232) aus wärmeleitendem Kunststoff oder Metall besteht, dass auf den Stift (232) ein Profilkörper (231) aufgesetzt ist und dass der Profilkörper (231) aus einem Erdalkalimetall, Calciumchlorid, Calciumchlorid, Magnesiumsulfat, Natriumhydroxid oder Calciumoxid hergestellt ist.

2. Heizvorrichtung nach Anspruch 1, **dadurch gekennzeichnet, dass** der Flüssigkeitsbehälter (220) mit Wasser gefüllt ist.

3. Verwendung einer Heizvorrichtung gemäß einem der Ansprüche 1 oder 2 für eine rauchfreie Zigarette.

4. Rauchfreie Zigarette mit einem hülsenförmigen Zigarettenkörper (100) und einem in dem Zigarettenkörper (100) befindlichen Depot (300), das mit Nikotin und einer Trägersubstanz gefüllt ist, **dadurch gekennzeichnet, dass** eine Heizvorrichtung (200) nach einem der Ansprüche 1 oder 2 vorgesehen ist, wobei die Heizvorrichtung (200) aus einem Flüssigkeitsbehälter (220), der mit einem Stoff gefüllt ist, und einem Reaktionselement (230), das aus einem Stoff hergestellt ist, besteht, wobei eine Vermischung dieser Stoffe eine exotherme Reaktion auslöst.

5. Rauchfreie Zigarette nach Anspruch 4, **dadurch gekennzeichnet, dass** eine Betätigungseinrichtung zum Auslösen der Heizvorrichtung vorgesehen ist.

6. Rauchfreie Zigarette nach Anspruch 5, **dadurch gekennzeichnet, dass** der Flüssigkeitsbehälter in einer Aufnahme der Betätigungseinrichtung angeordnet ist.

7. Rauchfreie Zigarette nach Anspruch 4, **dadurch gekennzeichnet, dass** der Flüssigkeitsbehälter (220) in dem Zigarettenkörper (100) verschiebbar angeordnet ist.

## Claims

1. Heating device for a smoke-free cigarette consisting of a liquid container (220), which is filled with a substance, and a reaction element (230), which is made of a substance, wherein a mixing of said substances causes an exothermic reaction and which are separated from one another by at least one destructible seal (223), wherein a pin (232) is provided at the reaction element (230), which interacts with the seal (223), **characterised in that** the pin (232) consists of heat-conducting plastic or metal, that a profile body (231) is placed on the pin (232) and that the profile body (231) is made of an alkaline earth metal, calcium chloride, calcium chloride, magnesium sulphate, sodium hydroxide or calcium oxide.

2. Heating device according to claim 1, **characterised in that** the liquid container (220) is filled with water.

3. The use of a heating device according to one of claims 1 or 2 for a smoke-free cigarette.

4. Smoke-free cigarette having a sleeve-shaped cigarette body (100) and a depot (300) located in the cigarette body (100), which is filled with nicotine and a carrier substance, **characterised in that** a heating device (200) according to one of claims 1 or 2 is provided, wherein the heating device (200) consists of a liquid container (220), which is filled with a substance, and a reaction element (230), which is made of a substance, wherein a mixing of said substances causes an exothermic reaction.

5. Smoke-free cigarette according to claim 4, **characterised in that** an actuation device for triggering the heating device is provided.

6. Smoke-free cigarette according to claim 5, **characterised in that** the liquid container is arranged in a receptacle of the actuation device.

7. Smoke-free cigarette according to claim 4, **characterised in that** the liquid container (220) is displaceably arranged in the cigarette body (100).

## Revendications

1. Dispositif chauffant de cigarette électronique, composé d'un réservoir de liquide (220) rempli d'une substance, et d'un élément de réaction (230) fabriqué dans une matière, sachant qu'un mélange de ces substances déclenche une réaction exotherme et qu'ils sont séparés l'un de l'autre par au moins un joint (223) destructible, sachant que contre l'élément de réaction (230) est prévu une tige (232) qui interagit avec le joint (223), **caractérisé en ce que** la tige (232) est en matière plastique thermoconductrice ou en métal, et que sur la tige (232) est posée un corps profilé (231) et **en ce que** le corps profilé (231) est fabriqué à partir d'un métal alcalino-terreux, de chlorure de calcium, de chlorure de calcium, de sulfate de magnésium, d'hydroxyde de sodium ou d'oxyde de calcium.

2. Dispositif chauffant selon la revendication 1, **caractérisé en ce que** le réservoir de liquide (220) est rempli d'eau.

3. Utilisation d'un dispositif chauffant selon la revendication 1 ou 2 pour une cigarette électronique.

4. Cigarette électronique composée d'un corps (100) de cigarette en forme de douille et d'une cavité (300), se trouvant dans le corps (100) de cigarette, cavité qui est remplie de nicotine et d'une substance porteuse, **caractérisée en ce qu'**un dispositif chauffant (200) selon l'une des revendications 1 ou 2 est prévu, sachant que le dispositif chauffant (200) se compose d'un réservoir (220) de liquide rempli d'une substance et d'un élément de réaction (230) fabriqué en un matériau, sachant qu'un mélange de ces substances déclenche une réaction exotherme.

5. Cigarette électronique selon la revendication 4, **caractérisée en ce qu'**un dispositif d'actionnement est prévu pour déclencher le dispositif chauffant.

6. Cigarette électronique selon la revendication 5, **caractérisée en ce que** le réservoir de liquide est disposé dans un logement du dispositif d'actionnement.

7. Cigarette électronique selon la revendication 4, **caractérisée en ce que** le réservoir de liquide (220) est disposé de sorte à coulisser dans le corps (100) de la cigarette.
